# EUROPEAN PATENT APPLICATION

(11) **EP 4 837 563 A2**
(43) Date of publication of application: **11.11.2026**
(21) Application number: 26168700.8
(22) Date of filing: 27.03.2026
(51) Int. Cl.: A61K 9/00, A61K 8/49, A61K 9/107, A61K 31/395, A61P 17/02, A61Q 9/00, A61Q 19/00

(54) **TRANSDERMAL ADMINISTRATION COMPOSITION OF SUSTAINED-RELEASE FORMULATION AND ACTIVE INGREDIENT DELIVERY SYSTEM CONTAINING ALLANTOIN MICRO-NEEDLE CRYSTAL**

(30) Priority: 14.04.2025 KR 20250048265
(71) Applicant: Amorepacific Corporation, Seoul 04386 (KR)
(72) Inventor: Kim, Suil, 17074 Yongin-si, Gyeonggi-do (KR); Lee, Eun Soo, 17074 Yongin-si, Gyeonggi-do (KR); Lee, Somi, 17074 Yongin-si, Gyeonggi-do (KR); Jang, Geunhyuk, 17074 Yongin-si, Gyeonggi-do (KR)
(74) Representative: Schön, Christoph

(57) **Abstract**

One aspect of the present disclosure relates to a transdermal administration composition of a sustained-release formulation containing an allantoin micro-needle crystal and an active ingredient delivery system, wherein the allantoin micro-needle crystal and a composition (regardless of type) containing the same have a sustained-release characteristic in which allantoin is gradually released and continuously absorbed into a stratum corneum and an epidermis layer by penetrating into skin, and can be utilized as an active ingredient delivery system for delivering various ingredients, specifically, an active ingredient that is oil-soluble or water-soluble into the skin, so that the allantoin itself or the active ingredient impregnated in the crystal is released in a sustained-release manner.

## Description

### BACKGROUND OF THE INVENTION

### Field of the Invention

The present specification relates to a transdermal administration composition of a sustained-release formulation and an active ingredient delivery system comprising an allantoin micro-needle crystal.

### Description of the Related Art

Sustained-release formulation refers to a formulation designed such that a drug or an active ingredient is slowly released into a body, and the sustained-release formulation is widely manufactured and sold in the pharmaceutical field.

On the other hand, in the cosmetic field, a cosmetic of a sustained-release formulation is not well manufactured. This is because, due to characteristics of the sustained-release formulation designed such that the active ingredient is gradually released over time, it is difficult to maintain stability of the formulation, a release rate varies depending on skin absorption characteristics and a skin barrier, and since the sustained-release formulation is made through a special technology and process, not only is designing the formulation complex, but also the sustained-release formulation, for which it is essential that structural stability be maintained, should have no deformation, but it is difficult to satisfy such requirements in the cosmetic field.

Meanwhile, recently, home-care skin management cosmetics utilizing a "micro-needle (fine needle)" technology, which is mainly utilized in dermatological beauty medical devices, are gaining popularity. Such cosmetics have established a selling point with an expectation of being able to experience efficacy obtainable through dermatological procedures by applying a dermatological procedure principle that provides a stinging and uncomfortable sensation. A core ingredient of most home-care skin management cosmetics utilizing the micro-needle technology is a micro-needle in which cosmetic ingredients such as a Centella asiatica extract are contained in a purified silica needle composed of animal-based raw materials, and it serves as a physical delivery system that physically helps skin penetration without a separate device, thereby inducing a purchase motivation in consumers, enabling intuitive experience of active ingredients, and serving to help absorption of other active ingredients.

However, spicule (hydrolyzed sponge), which is a main component of such a micro-needle, is an animal-based raw material and has a skeleton derived from a sponge, and thus about 99% is composed of silica. For such spicule, there are problems that it is too stinging to be used on sensitive skin after being applied to the skin, and since the needle is stuck in the skin, pain continues to remain over time unless it is washed off with water.

Accordingly, the present inventors performed research on a new material capable of exhibiting an effect similar to receiving a dermatological procedure by having a shape like a micro-needle and thus enabling skin penetration, while being able to solve disadvantages of the spicule, and capable of acting as a sustained-release formulation in which an effective ingredient or an active ingredient is gradually released, and as a result, completed the present invention.

### SUMMARY OF THE INVENTION

The present inventors performed research on a new material capable of exhibiting an effect similar to receiving a dermatological procedure by having a shape like a micro-needle and thus enabling skin penetration, while being able to solve disadvantages of the spicule, and capable of acting as a sustained-release formulation in which an effective ingredient or an active ingredient is gradually released, and as a result, confirmed that a crystal in which atypical allantoin is precipitated in a shape like a micro-needle can obtain such effects.

Therefore, in one aspect, an object of the present invention is to provide a new material that has a shape like a micro-needle while providing a user with a sensation like receiving a dermatological procedure when applied to skin and acting as a sustained-release formulation in which an effective ingredient is continuously released to the skin.

One aspect of the present disclosure provides a transdermal administration composition of a sustained-release formulation comprising an allantoin micro-needle crystal.

Another aspect of the present disclosure provides an active ingredient delivery system for delivering an active ingredient into skin, wherein the system comprises an allantoin micro-needle crystal, and the allantoin micro-needle crystal has one or more features of: an average length of a long axis of 5 to 500 µm; and a thickness of greater than 0 µm and 40 µm or less.

The allantoin micro-needle crystal and a composition (regardless of type) comprising the same according to one aspect of the present disclosure have a sustained-release characteristic in which the allantoin is gradually released and continuously absorbed into a stratum corneum and an epidermis layer by penetrating into the skin, and can be utilized as an active ingredient delivery system for delivering various ingredients, specifically, an active ingredient that is oil-soluble or water-soluble into the skin, so that the allantoin itself or the active ingredient impregnated in the crystal is released in a sustained-release manner, whereby an effect lasts for a long time and a stable effect is maintained, side effects such as skin irritation or allergic reactions are reduced, and furthermore, the active ingredient can be continuously delivered to a specific site, which is effective for improving a skin condition, and since a small amount of ingredients is continuously released, there is an excellent effect that the skin can be easily managed for a long time without a burden on a skin barrier.

### BRIEF DESCRIPTION OF THE DRAWINGS

FIG. 1 is a photograph of an allantoin micro-needle crystal according to an embodiment of the present invention.
FIG. 2A is a photograph of a cross-section of an untreated group to which nothing was treated after Nile red staining in an *ex vivo* stratum corneum residual evaluation experiment according to an embodiment of the present invention.
FIG. 2B is a photograph of a cross-section when 18 hours have elapsed after an allantoin powder, which is a control group, was applied to a skin biopsy tissue in the *ex vivo* stratum corneum residual evaluation experiment according to an embodiment of the present invention.
FIG. 2C is a photograph of a cross-section when 18 hours have elapsed after the allantoin micro-needle crystal according to an embodiment of the present invention was applied to a skin biopsy tissue in the *ex vivo* stratum corneum residual evaluation experiment according to an embodiment of the present invention.
FIG. 3A is a photograph of a cross-section of an untreated group to which nothing was treated after Nile red staining (left), and photographs of cross-sections when 2 hours (middle) and 8 hours (right) have respectively elapsed after an oil-in-water composition including the allantoin micro-needle crystal according to an embodiment of the present invention was applied to a skin biopsy tissue in the *ex vivo* stratum corneum residual evaluation experiment.
FIG. 3B is a graph showing a result of measuring fluorescence intensity detected from the oil-in-water composition including the allantoin micro-needle crystal according to an embodiment of the present invention over time.
FIG. 3C is a graph showing a result of evaluating skin absorption capability through relative fluorescence intensity according to time and a skin depth (z-depth)from the outermost part to the inside of a skin stratum corneum after the oil-in-water composition including the allantoin micro-needle crystal according to an embodiment of the present invention was applied to a skin biopsy tissue in the *ex vivo* stratum corneum residual evaluation experiment.
FIG. 4A is a photograph of a cross-section of an untreated group to which nothing was treated after Nile red staining (left), and photographs of cross-sections when 2 hours (middle) and 8 hours (right) have respectively elapsed after a liposome formulation composition including the allantoin micro-needle crystal according to an embodiment of the present invention was applied to a skin biopsy tissue in the *ex vivo* stratum corneum residual evaluation experiment.
FIG. 4B is a graph showing a result of measuring fluorescence intensity detected from the liposome formulation composition including the allantoin micro-needle crystal according to an embodiment of the present invention over time.
FIGS. 5A and 5B are graphs showing results of measuring skin absorption capability of each of the oil-in-water composition and the liposome formulation composition including the allantoin micro-needle crystal according to an embodiment of the present invention.
FIG. 6 is a photograph of a manufacturing process of an active ingredient delivery system in which retinol is impregnated into the allantoin micro-needle crystal according to an embodiment of the present invention.
FIGS. 7 and 8 are photographs of an allantoin micro-needle crystal as an active ingredient delivery system in which each of retinol (FIG. 7) and a Centella asiatica extract (FIG. 8) is impregnated into the allantoin micro-needle crystal according to an embodiment of the present invention.

### DETAILED DESCRIPTION OF THE INVENTION

In this specification, "acicular" or "needle" refers to a shape that is thin like a needle and has a sharp tip, and "crystal" refers to a crystalline solid that does not substantially include a fixed molar ratio of solvent molecules within a crystal lattice, that is, a crystalline solid that is not a solvate, wherein "acicular crystal" or "needle crystal" refers to a needle-shaped crystal.

In this specification, a transdermal administration composition comprises a cosmetic composition and a pharmaceutical composition. Hereinafter, the present invention will be described in detail.

In one aspect, the present invention provides a transdermal administration composition of a sustained-release formulation comprising an allantoin micro-needle crystal.

Allantoin (C₄H₆N₄O₃, molecular weight 158.116 g/mol, IUPAC name (2,5-dioxo-4-imidazolidinyl) urea, CAS No. 97-59-6, EC No. 202-592-8) according to one aspect of the present disclosure is an ingredient approved by the U.S. FDA as an over-the-counter (OTC) drug as an active ingredient for skin protection, and is known as a poorly soluble ingredient. The allantoin may be represented by the following [Chemical Formula 1].

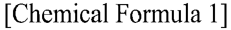

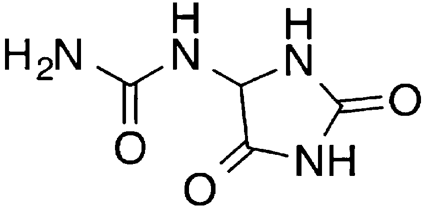

The allantoin crystal according to one aspect of the present disclosure has a micro-needle structure and thus may give a fine stimulus to skin when applied to the skin, thereby promoting absorption into the skin.

The allantoin micro-needle crystal according to one aspect of the present disclosure may have one or more features of: an average length of a long axis of 5 to 500 µm; and a thickness of greater than 0 µm and 40 µm or less.

The average length of the long axis of the allantoin micro-needle crystal according to one aspect of the present disclosure may be 5 to 500 µm. Specifically, the average length of the long axis of the allantoin micro-needle crystal may be 5 µm or more, 10 µm or more, 20 µm or more, 30 µm or more, 40 µm or more, 50 µm or more, 55 µm or more, 60 µm or more, 65 µm or more, 70 µm or more, 75 µm or more, 80 µm or more, 85 µm or more, 90 µm or more, 92 µm or more, 94 µm or more, 95 µm or more, 96 µm or more, 98 µm or more, 100 µm or more, 110 µm or more, 120 µm or more, 130 µm or more, 140 µm or more, 150 µm or more, 160 µm or more, 170 µm or more, 180 µm or more, 190 µm or more, 200 µm or more, 250 µm or more, 300 µm or more, 350 µm or more, 400 µm or more, or 450 µm or more, and may be 500 µm or less, 450 µm or less, 400 µm or less, 350 µm or less, 300 µm or less, 250 µm or less, 240 µm or less, 230 µm or less, 225 µm or less, 220 µm or less, 218 µm or less, 216 µm or less, 214 µm or less, 212 µm or less, 210 µm or less, 208 µm or less, 206 µm or less, 204 µm or less, 202 µm or less, 200 µm or less, 180 µm or less, 160 µm or less, 140 µm or less, 120 µm or less, 100 µm or less, 90 µm or less, 80 µm or less, 70 µm or less, or 60 µm or less. More specifically, the average length of the long axis of the allantoin micro-needle crystal may be 100 to 200 µm, and even more specifically, may be 150 to 200 µm.

The thickness of the allantoin micro-needle crystal according to one aspect of the present disclosure may be greater than 0 µm and 40 µm or less. Specifically, the thickness of the allantoin micro-needle crystal may be greater than 0 µm, 2 µm or more, 4 µm or more, 5 µm or more, 6 µm or more, 7 µm or more, 8 µm or more, 9 µm or more, 10 µm or more, 11 µm or more, 12 µm or more, 13 µm or more, 14 µm or more, 15 µm or more, 16 µm or more, 17 µm or more, 18 µm or more, 19 µm or more, 20 µm or more, 22 µm or more, 24 µm or more, 26 µm or more, 28 µm or more, 30 µm or more, or 35 µm or more, and may be 40 µm or less, 38 µm or less, 36 µm or less, 34 µm or less, 32 µm or less, 30 µm or less, 29 µm or less, 28 µm or less, 27 µm or less, 26 µm or less, 25 µm or less, 24 µm or less, 23 µm or less, 22 µm or less, 21 µm or less, 20 µm or less, 15 µm or less, 10 µm or less, or 8 µm or less. More specifically, the thickness of the allantoin micro-needle crystal may be 10 to 20 µm. The allantoin micro-needle crystal according to one aspect of the present disclosure has a thickness of greater than 0 µm and 40 µm or less, and thus may slightly penetrate a skin surface and pass through a stratum corneum.

The allantoin micro-needle crystal according to one aspect of the present disclosure may be one crystallized by precipitating atypical allantoin through an anti-solvent crystallization method.

The allantoin micro-needle crystal according to one aspect of the present disclosure may be soluble. The allantoin micro-needle crystal may be water-soluble or oil-soluble. The allantoin micro-needle crystal has an excellent solubility in a solvent and thus has a soluble characteristic, which has an effect of solving a problem of poor solubility of general allantoin powder.

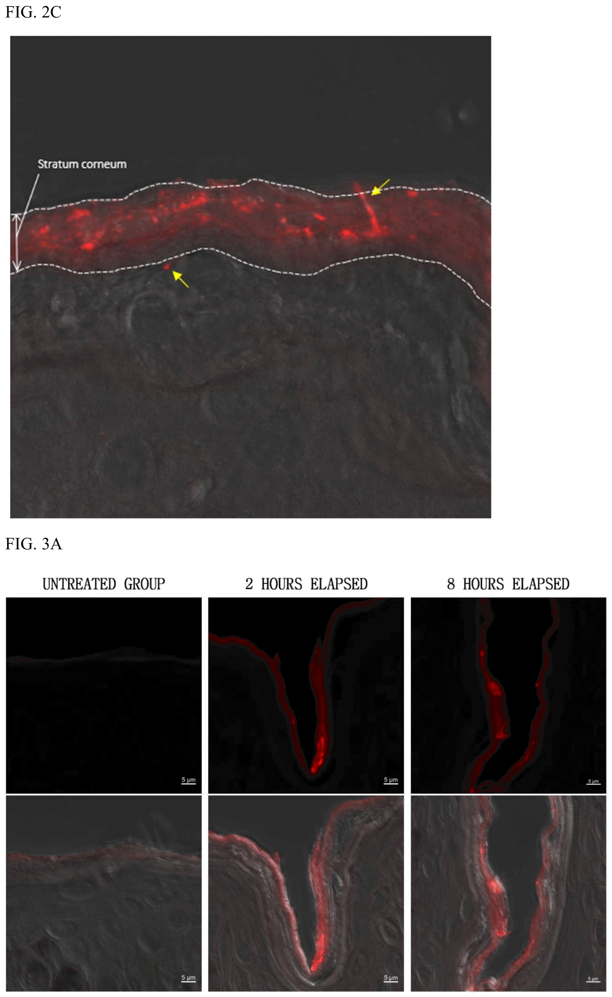

The allantoin micro-needle crystal according to one aspect of the present disclosure may penetrate into skin when applied to the skin. In addition, the allantoin micro-needle crystal according to one aspect of the present disclosure may exist inside a skin stratum corneum when 2 to 36 hours have elapsed after being applied to the skin. Specifically, the allantoin micro-needle crystal may exist inside a skin stratum corneum when 2 hours or more, 3 hours or more, 4 hours or more, 5 hours or more, 6 hours or more, 7 hours or more, 8 hours or more, 9 hours or more, 10 hours or more, 12 hours or more, 14 hours or more, 16 hours or more, 18 hours or more, 20 hours or more, 24 hours or more, 30 hours or more, or 36 hours or more have elapsed after being applied to the skin. According to an embodiment of the present invention, by having a micro-needle shape like a needle, it remains inside the stratum corneum even after much time has elapsed after being applied to the skin, and thus, simply by applying it to the skin, it may penetrate into the skin and be absorbed into the skin (see Experimental Example 1 and FIG. 2C).

The allantoin micro-needle crystal according to one aspect of the present disclosure may be dissolved in skin and absorbed into the skin after being applied to the skin. The process may be as follows: first, when applying the allantoin micro-needle crystal onto skin, the allantoin micro-needle crystal itself may be attached to a skin surface through direct application. Then, the applied allantoin micro-needle crystal has a thickness of greater than 0 µm and 40 µm or less, specifically 5 to 40 µm, and thus slightly penetrates a skin surface and may pass through a skin stratum corneum. In this case, an allantoin ingredient may be immediately delivered into the skin, and due to the micro-needle structure, a fine stimulus is applied to the skin, so that absorption into the skin can be further promoted. Thereafter, when rolling or rubbing the skin surface with a hand or a finger about 10 times, contact of allantoin micro-needle(fine arcicular) crystals with the skin surface is maximized, which can help penetration to be smoothly performed. The rolling or rubbing motion may promote dissolution of the allantoin micro-needle crystal in the skin and absorption into the skin, and a micro-circulation improvement effect by fine stimulation may also be expected. Further, the allantoin micro-needle crystal penetrated into the skin or the skin stratum corneum is slowly dissolved while contacting with moisture in the skin, and allantoin is released, which may be performed within 2 to 36 hours after application. A specific absorption process is first absorbed into a stratum corneum (Keratin Layer), which reacts with subcutaneous moisture to be gradually dissolved, and some ingredients gradually reach even an epidermis layer (Epidermis) to be secondarily absorbed. In general, the allantoin micro-needle crystal is mostly dissolved and absorbed within the stratum corneum.

The allantoin micro-needle crystal according to one aspect of the present disclosure may be dissolved and absorbed in skin when repeatedly rolled 10 times with a weight of 0.5 to 1.0 kg/cm² after being applied to the skin. The allantoin micro-needle crystal may be dissolved and absorbed in skin when 2 hours or more, 3 hours or more, 4 hours or more, 5 hours or more, 6 hours or more, 7 hours or more, 8 hours or more, 9 hours or more, 12 hours or more, 15 hours or more, 18 hours or more, 21 hours or more, 24 hours or more, 27 hours or more, 30 hours or more, 33 hours or more, or 36 hours or more have elapsed after being applied to the skin, but dissolution and absorption into the skin may be promoted by rolling with a hand or a finger. Specifically, the allantoin micro-needle crystal may be dissolved in skin and absorbed into a skin stratum corneum when repeatedly rolled 1 time, 2 times, 3 times, 4 times, 5 times, 6 times, 7 times, 8 times, 9 times, or 10 times with a weight of 0.5 kg/cm² or more, 0.6 kg/cm² or more, 0.7 kg/cm² or more, 0.8 kg/cm² or more, or 0.9 kg/cm² or more, and a weight of 1.0 kg/cm² or less, 0.9 kg/cm² or less, 0.8 kg/cm² or less, 0.7 kg/cm² or less, or 0.6 kg/cm² or less after being applied to the skin. The rolling or rubbing motion promotes dissolution of the allantoin micro-needle crystal and absorption into the skin, and a micro-circulation improvement effect by fine stimulation may also be expected.

A composition according to one aspect of the present disclosure may be a sustained-release formulation. The sustained-release formulation is a formulation that uniformly and continuously releases an effective ingredient or an active ingredient included in the composition by using a sustained-release base, and an effect can be continuously maintained by allowing absorption of the effective ingredient or the active ingredient to be continuously performed in a living body.

A composition according to one aspect of the present disclosure may be for transdermal administration.

The allantoin micro-needle crystal according to one aspect of the present disclosure may comprise 3 to 16 wt% of the allantoin based on a total weight of the allantoin micro-needle crystal. Specifically, the allantoin micro-needle crystal may comprise 3 wt% or more, 3.5 wt% or more, 4 wt% or more, 4.2 wt% or more, 4.4 wt% or more, 4.6 wt% or more, 4.8 wt% or more, 5 wt% or more, 5.2 wt% or more, 5.4 wt% or more, 5.6 wt% or more, 5.8 wt% or more, 6 wt% or more, 7 wt% or more, 8 wt% or more, 9 wt% or more, 10 wt% or more, 11 wt% or more, 12 wt% or more, or 14 wt% or more of the allantoin based on the total weight of the allantoin micro-needle crystal, and may comprise 16 wt% or less, 15 wt% or less, 14 wt% or less, 13 wt% or less, 12 wt% or less, 11 wt% or less, 10 wt% or less, 9 wt% or less, 8.8 wt% or less, 8.6 wt% or less, 8.4 wt% or less, 8.2 wt% or less, 8 wt% or less, 7.8 wt% or less, 7.6 wt% or less, 7.4 wt% or less, 7.2 wt% or less, 7 wt% or less, 6 wt% or less, 5 wt% or less, or 4 wt% or less of the allantoin based on the total weight of the allantoin micro-needle crystal. More specifically, the allantoin micro-needle crystal may comprise 4 to 9 wt% of the allantoin based on the total weight of the allantoin micro-needle crystal.

The allantoin micro-needle crystal according to one aspect of the present disclosure may further comprise betaine. The allantoin micro-needle crystal may crystallize allantoin in a micro-needle shape with higher purity and yield when including betaine.

The allantoin micro-needle crystal according to one aspect of the present disclosure may comprise 9 to 72 wt% of the betaine based on the total weight of the allantoin micro-needle crystal. Specifically, the allantoin micro-needle crystal may comprise 9 wt% or more, 10 wt% or more, 12 wt% or more, 14 wt% or more, 16 wt% or more, 18 wt% or more, 20 wt% or more, 22 wt% or more, 24 wt% or more, 26 wt% or more, 28 wt% or more, 30 wt% or more, 35 wt% or more, 36 wt% or more, 40 wt% or more, 50 wt% or more, or 60 wt% or more of the betaine based on the total weight of the allantoin micro-needle crystal, and may comprise 72 wt% or less, 70 wt% or less, 60 wt% or less, 50 wt% or less, 40 wt% or less, 38 wt% or less, 36 wt% or less, 34 wt% or less, 32 wt% or less, 30 wt% or less, 28 wt% or less, 26 wt% or less, 24 wt% or less, 22 wt% or less, 20 wt% or less, 19 wt% or less, 18 wt% or less, 16 wt% or less, 14 wt% or less, 12 wt% or less, or 10 wt% or less of the betaine based on the total weight of the allantoin micro-needle crystal. More specifically, the allantoin micro-needle crystal may comprise 12 to 36 wt% of the betaine based on the total weight of the allantoin micro-needle crystal.

A composition according to one aspect of the present disclosure may comprise 3 to 12 wt% of the allantoin micro-needle crystal based on a total weight of the composition. Specifically, the composition may comprise 3 wt% or more, 3.5 wt% or more, 4 wt% or more, 4.2 wt% or more, 4.4 wt% or more, 4.6 wt% or more, 4.8 wt% or more, 5 wt% or more, 5.2 wt% or more, 5.4 wt% or more, 5.6 wt% or more, 5.8 wt% or more, 6 wt% or more, 7 wt% or more, 8 wt% or more, 9 wt% or more, or 10 wt% or more of the allantoin micro-needle crystal based on the total weight of the composition, and may comprise 12 wt% or less, 11 wt% or less, 10 wt% or less, 9 wt% or less, 8.8 wt% or less, 8.6 wt% or less, 8.4 wt% or less, 8.2 wt% or less, 8 wt% or less, 7.8 wt% or less, 7.6 wt% or less, 7.4 wt% or less, 7.2 wt% or less, 7 wt% or less, 6 wt% or less, 5 wt% or less, or 4 wt% or less of the allantoin micro-needle crystal based on the total weight of the composition. More specifically, the composition may comprise 4 to 9 wt% of the allantoin micro-needle crystal based on a total weight of the composition.

The composition according to one aspect of the present disclosure may be a composition for external application to skin.

The composition according to one aspect of the present disclosure may be a composition for transdermal administration.

The composition according to one aspect of the present disclosure may be a cosmetic composition or a pharmaceutical composition.

The cosmetic composition according to one aspect of the present disclosure may be for exfoliation, skin moisturizing, skin wound healing, promoting skin regeneration, or skin protection against irritation.

In the composition according to one aspect of the present disclosure, an appearance of the cosmetic composition may contain a cosmetically or dermatologically acceptable medium or base. This includes all formulations suitable for topical application, and may be provided, for example, in a form of a solution, a gel, a solid, a paste anhydrous product, an emulsion obtained by dispersing an oil phase in an aqueous phase, a suspension, a microemulsion, a microcapsule, a microgranule, or an ionic (liposome) and non-ionic vesicular dispersant, or in a form of a cream, a toner, a lotion, a powder, an ointment, a spray, or a concealer stick. These compositions may be manufactured according to conventional methods in the art. The composition according to the present disclosure may also be used in a form of a foam or in a form of an aerosol composition further containing a compressed propellant.

In the composition according to one aspect of the present disclosure, the cosmetic composition is not particularly limited in its formulation, and may be formulated into cosmetics such as, for example, softening cosmetic water, astringent cosmetic water, nourishing cosmetic water, nourishing cream, massage cream, essence, eye cream, eye essence, cleansing cream, cleansing foam, cleansing water, a cleansing tissue including the cosmetic composition, a facial pack, a powder, a body lotion, a body cream, a body oil, and a body essence.

In the composition according to one aspect of the present disclosure, when the formulation of the cosmetic composition is a paste, a cream, or a gel, animal fiber, vegetable fiber, wax, paraffin, starch, tragacanth, cellulose derivative, polyethylene glycol, silicone, bentonite, silica, talc, or zinc oxide, etc. may be used as a carrier component.

In the composition according to one aspect of the present disclosure, when the formulation of the cosmetic composition is a powder or a spray, lactose, talc, silica, aluminum hydroxide, calcium silicate, or polyamide powder may be used as a carrier component, and particularly in a case of a spray, a propellant such as chlorofluorohydrocarbon, propane/butane, or dimethyl ether may be additionally included.

In the composition according to one aspect of the present disclosure, when the formulation of the cosmetic composition is a solution or an emulsion, a solvent, a solubilizing agent, or an emulsifying agent is used as a carrier component, such as water, ethanol, isopropanol, ethyl carbonate, ethyl acetate, benzyl alcohol, benzyl benzoate, propylene glycol, 1,3-butyl glycol oil, glycerol aliphatic ester, polyethylene glycol, or fatty acid ester of sorbitan.

In the composition according to one aspect of the present disclosure, when the formulation of the cosmetic composition is a suspension, a liquid diluent such as water, ethanol, or propylene glycol, a suspending agent such as ethoxylated isostearyl alcohol, polyoxyethylene sorbitol ester, and polyoxyethylene sorbitan ester, microcrystalline cellulose, aluminum metahydroxide, bentonite, agar, or tragacanth, etc. may be used as a carrier component.

In the composition according to one aspect of the present disclosure, a functional additive and ingredients included in a general cosmetic composition may be further included in the cosmetic composition. The functional additive may include a component selected from the group consisting of water-soluble vitamins, oil-soluble vitamins, polymeric peptides, polymeric polysaccharides, squalane, sphingolipids, and seaweed extract.

In the composition according to one aspect of the present disclosure, in addition to the functional additive, ingredients included in a general cosmetic composition may be blended into the cosmetic composition as necessary. As other blending ingredients included, fat and oil components, moisturizers, emollients, emulsifiers, organic and inorganic pigments, organic powders, ultraviolet absorbers, antiseptics, bactericides, antioxidants, plant extracts, pH adjusters, alcohols, colorants, fragrances, blood circulation promoters, cooling agents, antiperspirants, purified water, and the like may be mentioned.

The pharmaceutical composition according to one aspect of the present disclosure may be a pharmaceutical composition for skin wound healing.

The pharmaceutical composition according to one aspect of the present disclosure may be provided in all formulations suitable for topical application. For example, it may be administered by oral, transdermal, intravenous, intramuscular, or subcutaneous injection, and specifically, may be administered transdermally. The pharmaceutical composition may be a solution for external application to skin, a suspension, an emulsion, a gel, a patch, or a spray, but is not limited thereto. The formulation may be easily manufactured by conventional methods in the art, and surfactants, excipients, hydrating agents, emulsification promoters, suspending agents, salts or buffers for osmotic pressure control, coloring agents, flavoring agents, stabilizers, antiseptics, preservatives, or other commonly used adjuvants may be appropriately used.

An active ingredient of the pharmaceutical composition according to one aspect of the present disclosure will vary depending on age, sex, body weight, pathological condition and severity thereof, administration route, or judgment of a prescriber. The determination of an applied dosage based on such factors is within a level of a person skilled in the art, and a daily usage amount thereof may be, for example, 0.1 mg/kg/day to 5000 mg/kg/day, and more specifically may be 50 mg/kg/day to 500 mg/kg/day, but is not limited thereto.

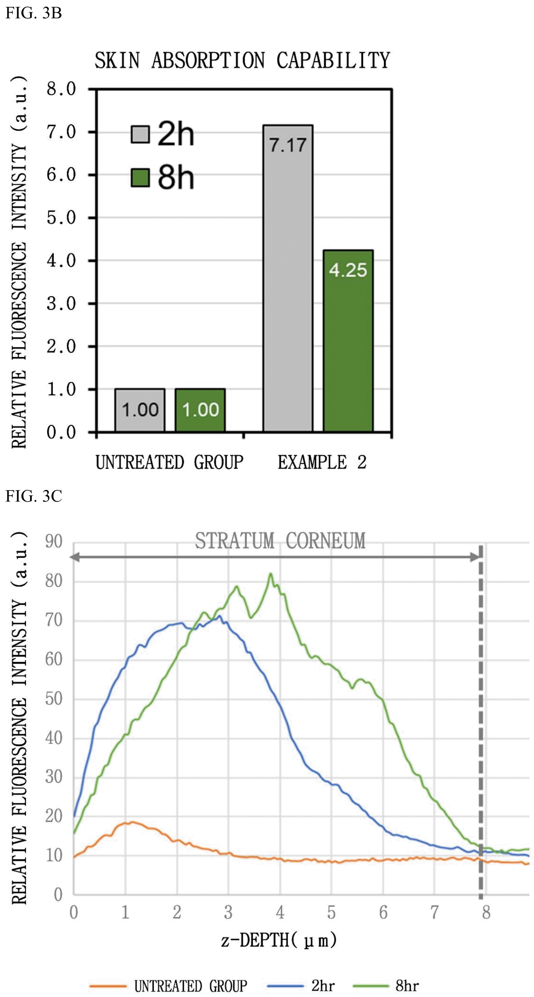

The composition according to one aspect of the present disclosure may be a composition of an oil dispersion, a water dispersion, a water-in-oil, an oil-in-water, or a multiple emulsion type. According to an embodiment of the present invention, a composition comprising the allantoin micro-needle crystal according to one aspect of the present disclosure, regardless of its type, including a water-in-oil type (e.g., a liposome formulation) or an oil-in-water type, penetrates into a skin stratum corneum after being applied to skin, and thereafter, allantoin, which is an active ingredient, is gradually released for a long time and continuously absorbed into the stratum corneum and an epidermis layer, thereby having a sustained-release characteristic (see Experimental Example 2 and FIGS. 3 to 5).

In another aspect, the present invention provides an active ingredient delivery system comprising the allantoin micro-needle crystal for delivering an active ingredient into skin. In the transdermal administration composition of the sustained-release formulation comprising the allantoin micro-needle crystal, a description of the allantoin micro-needle crystal also applies to the active ingredient delivery system.

The active ingredient delivery system according to one aspect of the present disclosure comprises an allantoin micro-needle crystal, and the allantoin micro-needle crystal has one or more features of: an average length of a long axis of 5 to 500 µm; and a thickness of greater than 0 µm and 40 µm or less.

The active ingredient delivery system according to one aspect of the present disclosure may further comprise an active ingredient within the allantoin micro-needle crystal. Specifically, the active ingredient may be a water-soluble or oil-soluble active ingredient. More specifically, the active ingredient may be one or more selected from the group consisting of retinol, vitamin C (ascorbic acid), niacinamide, coenzyme Q10, resveratrol, glutathione, ceramide, azelaic acid, astaxanthin, epigallocatechin gallate (EGCG), phytosterols, Centella asiatica extract (Centella asiatica triterpene (ASIATICOSIDE*MADECASSIC ACID*ASIATIC ACID)), salicylic acid, oleanolic acid, nicotinamide mononucleotide, hyaluronic acid, L-carnitine, vitamin B complex, amino acids, acetyl hexapeptide-8, panthenol (vitamin B5), ubiquinone, hydrolyzed ginseng saponins, *Panax ginseng* root extract, *Panax ginseng* seed oil, *Panax ginseng* berry extract, peony root extract, Polygonatum rhizome extract, lotus extract, Lilium lancifolium bulb extract, and mixtures thereof.

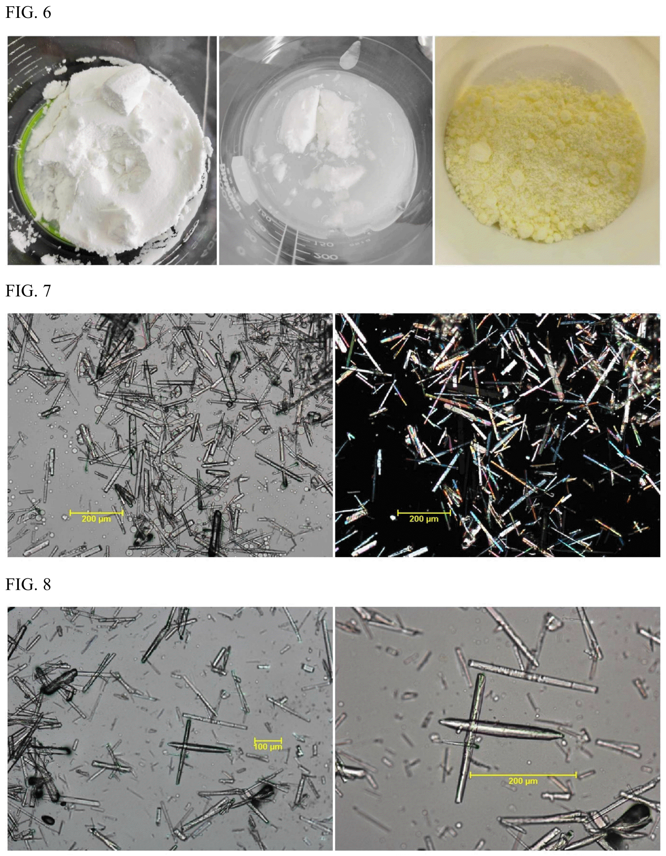

According to an embodiment of the present invention, each of retinol, which is a representative oil-soluble component, and a Centella asiatica extract, which is a representative component dissolved in polyol as a representative water-soluble component, is impregnated into the allantoin micro-needle crystal, and thus, the allantoin micro-needle crystal according to one aspect of the present disclosure can be utilized as an active ingredient delivery system that delivers various components, specifically, an oil-soluble or water-soluble active ingredient into skin by crystallizing allantoin, which was poorly soluble, by changing it to be soluble (see Experimental Example 3 and FIGS. 6 and 8).

In another aspect, the present invention may relate to a method for exfoliation, skin moisturizing, skin wound healing, promoting skin regeneration, or skin protection against irritation, comprising transdermally administering a composition of a sustained-release formulation comprising an allantoin micro-needle crystal to a subject in need of exfoliation, skin moisturizing, skin wound healing, promoting skin regeneration, or skin protection against irritation.

In another aspect, the present invention may relate to a use of an allantoin micro-needle crystal for manufacturing a pharmaceutical composition for exfoliation, skin moisturizing, skin wound healing, promoting skin regeneration, or skin protection against irritation.

In another aspect, the present invention may relate to a use of an allantoin micro-needle crystal for manufacturing a cosmetic composition for exfoliation, skin moisturizing, skin wound healing, promoting skin regeneration, or skin protection against irritation.

In another aspect, the present invention may relate to a use of an allantoin micro-needle crystal for manufacturing a food composition for exfoliation, skin moisturizing, skin wound healing, promoting skin regeneration, or skin protection against irritation.

In another aspect, the present invention may relate to a use of an allantoin micro-needle crystal for exfoliation, skin moisturizing, skin wound healing, promoting skin regeneration, or skin protection against irritation.

The present disclosure is further described by the following embodiments which do not limit the scope of the claims.

Embodiment 1. A transdermal administration composition of a sustained-release formulation comprising an allantoin micro-needle crystal.

Embodiment 2. The transdermal administration composition of Embodiment 1, wherein the allantoin micro-needle crystal has one or more features of:
an average length of a long axis of 5 to 500 µm; and
a thickness of greater than 0 and 40 µm or less.

Embodiment 3. The transdermal administration composition of Embodiment 1 or 2, wherein the allantoin micro-needle crystal is soluble.

Embodiment 4. The transdermal administration composition of any one of Embodiments 1 to 3, wherein the allantoin micro-needle crystal penetrates into skin when applied to the skin.

Embodiment 5. The transdermal administration composition of any one of Embodiments 1 to 4, wherein the allantoin micro-needle crystal exists inside a skin stratum corneum when 2 to 36 hours have elapsed after being applied to skin.

Embodiment 6. The transdermal administration composition of any one of Embodiments 1 to 5, wherein the allantoin micro-needle crystal is dissolved and absorbed in skin when repeatedly rolled 10 times with a weight of 0.5 to 1.0 kg/cm2 after being applied to the skin.

Embodiment 7. The transdermal administration composition of any one of Embodiments 1 to 6, wherein the allantoin micro-needle crystal further comprises betaine.

Embodiment 8. The transdermal administration composition of any one of Embodiments 1 to 7, wherein the transdermal administration composition is a cosmetic composition for exfoliation, skin moisturizing, skin wound healing, promoting skin regeneration, or skin protection against irritation.

Embodiment 9. The transdermal administration composition of any one of Embodiments 1 to 8, wherein the transdermal administration composition is a cosmetic composition for exfoliation, skin moisturizing, improvement of damaged skin, promoting skin regeneration, or skin protection against irritation.

Embodiment 10.The transdermal administration composition of any one of Embodiments 1 to 9, wherein the transdermal administration composition is a pharmaceutical composition for skin wound healing.

Embodiment 11. The transdermal administration composition of any one of Embodiments 1 to 10, wherein the transdermal administration composition is a composition of an oil dispersion, a water dispersion, a water-in-oil, an oil-in-water, or a multiple emulsion type.

Embodiment 12. An active ingredient delivery system for delivering an active ingredient into skin, wherein the active ingredient system comprises an allantoin micro-needle crystal, and the allantoin micro-needle crystal has one or more features of:
an average length of a long axis of 5 to 500 µm; and
a thickness of greater than 0 µm and 40 µm or less.

Embodiment 13. The active ingredient delivery system of Embodiment 12, wherein the active ingredient delivery system further comprises an active ingredient within the allantoin micro-needle crystal.

Embodiment 13. The active ingredient delivery system of Embodiment 11 or 12, wherein the active ingredient is water-soluble or oil-soluble.

Hereinafter, configurations and effects of the present invention will be described in more detail through Examples and Experimental Examples. However, the following Examples and Experimental Examples are provided only for the purpose of illustration to help understanding of the present invention, and the category and scope of the present invention are not limited thereby.

### [Experimental Example 1] Confirmation of skin penetration and long-term absorption capability of allantoin micro-needle crystal

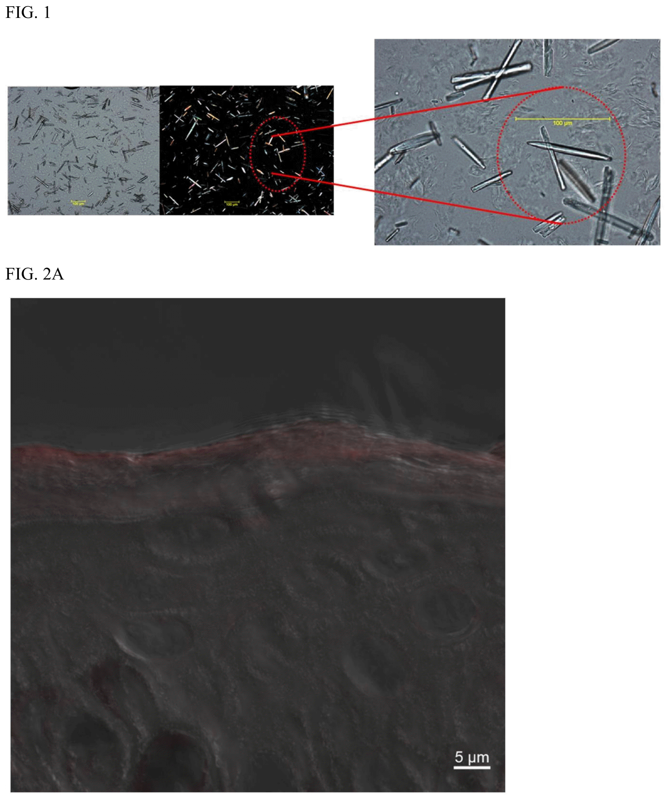

An allantoin micro-needle crystal of Example 1 was manufactured by dissolving 90g of allantoin in a powder form (allantoin (C₄H₆N₄O₃, molecular weight 158.116 g/mol, IUPAC name (2,5-dioxo-4-imidazolidinyl) urea, CAS No. 97-59-6, EC No. 202-592-8) (Clariant Produkte (Deutschland) GmbH Co., ALLANTOIN Premium)) along with 30 g of urea and 270 g of betaine in 600 g of glycerin by using a Deep Eutectic solvent condition through an anti-solvent crystallization method, adding water thereto, and then precipitating the same (FIG. 1). The allantoin micro-needle crystal has an acicular shape having an average length of a long axis of 150 to 200 µm and a thickness of 5 to 40 µm.

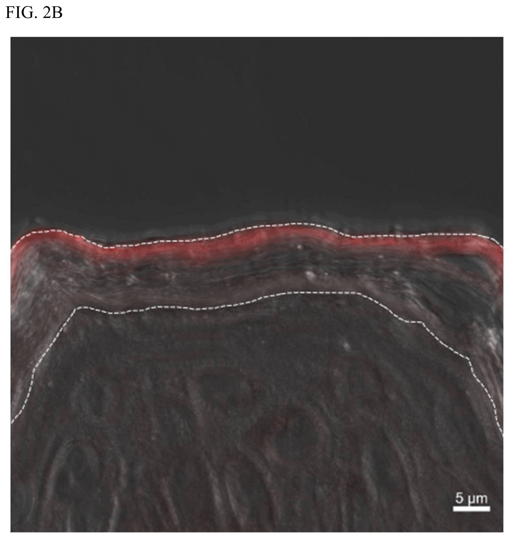

In order to confirm a skin absorption capability of the allantoin micro-needle crystal of Example 1, an *ex vivo* stratum corneum residual evaluation experiment was performed. After the allantoin micro-needle crystal of Example 1 stained with Nile-red (Sigma Aldrich Co., 72485-100mg) was applied onto an epidermis of an *ex vivo* skin biopsy tissue (Genoskin Co., NativeSkin^{™} #NSA20), which is a skin biopsy tissue with a diameter of 20 mm collected from an abdomen of a 54-year-old female, a cross-section of the skin biopsy tissue when 18 hours have elapsed was photographed, and an image thereof is as shown in FIG. 2C. As an untreated group, a cross-section of a skin biopsy tissue in which only fluorescence staining was performed without treating both the allantoin micro-needle crystal and the allantoin powder was photographed, and an image thereof is as shown in FIG. 2A. In addition, as a control group, an allantoin powder (Clariant Produkte (Deutschland) GmbH Co., ALLANTOIN Premium) was applied to a skin biopsy tissue in the same manner and a cross-section thereof was photographed, and an image thereof is as shown in FIG. 2B. In this case, a specific experimental process is as follows.

### Fluorescence Staining

For fluorescence staining, a Nile-red fluorescence staining reagent was dissolved in a DMSO solution to reach a concentration of 1 mg/mL, and to stain the allantoin micro-needle crystal or the allantoin powder, respectively, Nile-red and the raw material powder were mixed at a volume ratio of 5:1000, and then stained by being left at room temperature for 1 hour. Then, samples were prepared by dissolving the allantoin micro-needle crystal or the allantoin powder in DI water to reach a final concentration of 5%.

### Sample Treatment

On the day the skin biopsy tissue arrived, 2 mL of a tissue culture medium was added, and stabilization and culture were performed in an incubator at 37°C and 5% CO₂ for one day. The next day, 20 µL of the allantoin micro-needle crystal or the powder solution stained with Nile-red was dropped onto an epidermis, and then rubbed with a silicone rubber. After being air-dried in air with a thermomixer at 37°C for 1 hour in a clean bench, additional culture was performed for 17 hours in each incubator at 37°C and 5% CO₂. When 18 hours have elapsed after application, the epidermis was washed with deionized water (DI water), and then fixed with a formalin solution. After preparing frozen section slides with the fixed skin tissue, observation was performed with a Confocal laser scanning microscopy (Carl Zeiss Co., model name: LSM980).

### Microscope Measurement Conditions

For observation of a skin tissue cross-section, images for a Nile-red fluorescence signal and a Bright field signal were obtained, three for each group, using a 40X objective lens under conditions of an excitation wavelength of 561 nm and an emission wavelength of 600 to 700 nm.

As shown in FIGS. 2A to 2C, Nile-red is a lipophilic fluorescent substance and is separated in water, and thus cannot penetrate a stratum corneum (FIG. 2A), whereas the allantoin powder, which is a control group, stays at the outermost part of the stratum corneum and thus failed to penetrate skin even after much time has elapsed after being applied to the skin (FIG. 2B). However, the allantoin micro-needle crystal of Example 1 moved deeply into the inside from a surface of the stratum corneum, and as the micro-needle crystals inside the stratum corneum were fixed and dissolved, allantoin, which is an active ingredient, was gradually released and continuously acted within the stratum corneum and an epidermis layer (FIG. 2C). Particularly, it was confirmed that for the crystal of Example 1, even after 18 hours have elapsed from application, the gradually released ingredients stay in the stratum corneum for a long time and can exert effects possessed by allantoin. It was confirmed that since the allantoin crystal has an acicular shape, it easily penetrates into skin and thus can be absorbed into the skin.

Through this, it could be seen that the allantoin crystal according to one aspect of the present disclosure has a micro-needle shape like a needle, so that simply by applying it to skin, it penetrates into the skin, is fixed and dissolved in a skin stratum corneum, and allantoin, which is an active ingredient, is gradually released and can be continuously absorbed and act within the stratum corneum and an epidermis layer.

### [Experimental Example 2] Confirmation of sustained-release characteristic of composition including allantoin micro-needle crystal

Through the Experimental Example 1, it was confirmed that the allantoin micro-needle crystal can penetrate into skin and be absorbed in the skin for a long time, and thus an *ex vivo* stratum corneum absorption capability experiment over time was performed to confirm whether a composition including the same has a sustained-release characteristic. The in vitro skin (ex vivo skin) is an in vitro skin (ex vivo skin) biopsy tissue (Genoskin Co., NativeSkin^{™} #NSA20, Lot No. 20250210.02, Fitzpatrick classification 3), which is a skin biopsy tissue collected from an abdomen of a 40-year-old female.

### [Experimental Example 2-1] Confirmation of sustained-release characteristic of oil-in-water composition including allantoin micro-needle crystal

First, a Nile-red (Sigma Aldrich Co., 72485-100mg) fluorescence staining reagent was dissolved in a DMSO solution to reach a concentration of 1 mg/mL, and then mixed with the allantoin micro-needle crystal of Example 1 to reach a concentration of 2% (v/w) (Nile-red concentration: 20 µg/mL). Then, as an oil-in-water composition including the allantoin micro-needle crystal, in order to manufacture a composition of an essence formulation (Example 2), the allantoin micro-needle crystal of Example 1 and the essence formulation (specifically, purified water, butylene glycol, dipropylene glycol, glycerin, propylene glycol, 1,2-hexanediol, caprylic/capric triglyceride, ammonium acryloyldimethyltaurate/VP copolymer, polyglyceryl-6 caprylate, polyglyceryl-4 caprate, hydrogenated lecithin, cholesterol, sodium methyl stearoyl taurate, ethylhexylglycerin, and tocopherol) were mixed at a volume ratio of 50:950 (Nile-red concentration: 1 µg/mL). The composition of the essence formulation was manufactured by mixing generally used ingredients according to a method generally used in a cosmetic industry. Then, staining was performed by being left at room temperature for 1 hour, and a sample was prepared by dissolving the same in DI water so that the allantoin micro-needle crystal reached a final concentration of 5%.

Thereafter, on the day the skin biopsy tissue arrived, 2 mL of a tissue culture medium was added, and stabilization and culture were performed in an incubator at 37°C and 5% CO₂ for one day. The next day, 20 µL of the oil-in-water composition of Example 2 stained with Nile-red was dropped onto the epidermis, and then rubbed with a silicone rubber (Nile-red concentration: 1 µg/mL). After being air-dried in air with a thermomixer at 37°C for 1 hour in a clean bench, additional culture was performed for 1 hour and 7 hours, respectively, in each incubator at 37°C and 5% CO₂. When 1 hour or 7 hours have elapsed after application, the epidermis was washed with deionized water (DI water), and then fixed with a formalin solution. After preparing frozen section slides with the fixed skin tissue, observation was performed with a Confocal laser scanning microscopy (Carl Zeiss Co., model name: LSM980).

For observation of a skin tissue cross-section, images for a Nile-red fluorescence signal and a Bright field signal were obtained, three for each group, using a 40X objective lens under conditions of an excitation wavelength of 561 nm and an emission wavelength of 600 to 700 nm.

As a result, cross-sections of the skin biopsy tissue when 2 hours and 8 hours have respectively elapsed from application of the oil-in-water composition including the allantoin micro-needle crystal of Example 2 were photographed (FIG. 3A), and a relative fluorescence intensity (a.u.) was measured therefrom to evaluate a skin absorption capability (FIG. 3B). As an untreated group, a cross-section of a skin biopsy tissue in which only fluorescence staining was performed without treating both the allantoin micro-needle crystal and the allantoin powder was photographed and a relative fluorescence intensity was measured.

As shown in FIGS. 3A and 3B, the oil-in-water composition of Example 2 was rapidly absorbed into skin when 2 hours have elapsed after application to show a fluorescence intensity of 7.17 a.u., and showed a fluorescence intensity of as much as 4.25 a.u. even when 8 hours have elapsed after application. That is, it was confirmed that the composition of Example 2 stays in a skin stratum corneum for a long time.

Additionally, as a result of evaluating a skin absorption capability through a relative fluorescence intensity according to a skin depth (z-depth) and time from the outermost part to the inside of the skin stratum corneum based on the results (FIG. 3C), it can be inferred that in the oil-in-water composition of Example 2, the allantoin micro-needle crystal exists inside the stratum corneum even when 2 hours to 8 hours have elapsed, and as time passes, a median value of the z-depth moves to the right, so the allantoin micro-needle crystal moves toward the inside of skin, and it was confirmed that allantoin is continuously released inside the stratum corneum for 8 hours after application, and thus the oil-in-water composition of Example 2 has a sustained-release characteristic.

### [Experimental Example 2-2] Confirmation of sustained-release characteristic of water-in-oil composition including allantoin micro-needle crystal

Through the Experimental Example 2-1, the sustained-release characteristic of the oil-in-water composition including the allantoin micro-needle crystal was confirmed, and thus the same experiment as in the Experimental Example 2-1 was performed to confirm whether a water-in-oil composition also has a sustained-release characteristic.

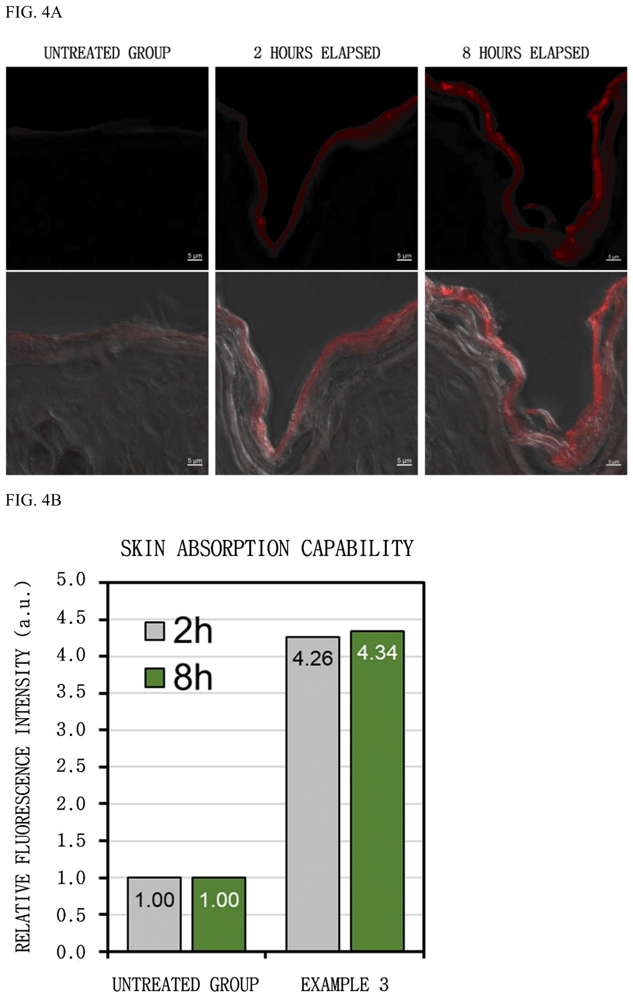

However, in this Experimental Example 2-2, a water-in-oil composition (Example 3) was used instead of the oil-in-water composition of Example 2. Specifically, the water-in-oil composition is a liposome formulation and was manufactured according to a method generally used in a cosmetic industry, and results thereof are as shown in FIGS. 4A and 4B.

As shown in FIGS. 4A and 4B, the liposome formulation composition of Example 3 showed similar fluorescence intensities of 4.26 a.u. and 4.34 a.u. when 2 hours and 8 hours have elapsed, respectively, and thus it was confirmed that the composition of Example 3 stays in a skin stratum corneum for a long time and is continuously absorbed into skin.

### [Experimental Example 2-3] Confirmation of sustained-release characteristic of oil-in-water and liposome formulation compositions including allantoin acicular crystals

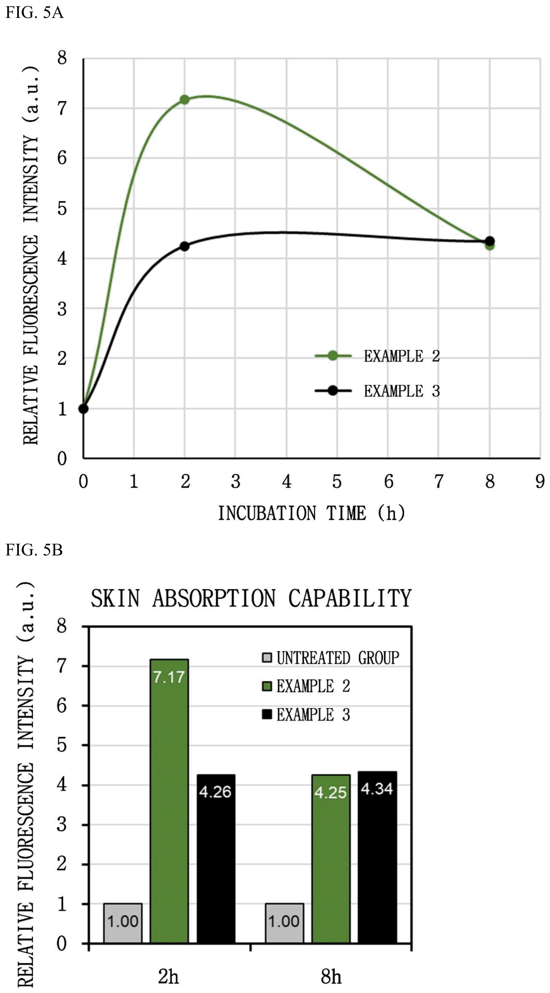

Based on the results of the Experimental Examples 2-1 and 2-2, skin absorption capabilities of the oil-in-water and liposome formulation compositions including the allantoin acicular crystals of Example 1 were compared (FIGS. 5A and 5B).

As shown in FIG. 5A, it was confirmed that the oil-in-water composition of Example 2 has a faster skin absorption rate and a more excellent skin penetration capability compared to the liposome formulation composition of Example 3. In addition, as can be seen from FIG. 5B, both the oil-in-water composition of Example 2 and the water-in-oil composition of Example 3 showed high fluorescence intensities in a skin stratum corneum even when 2 hours and 8 hours have elapsed after application, so that it could be seen that after the allantoin micro-needle crystal in the composition penetrated into the skin stratum corneum, allantoin was continuously released, and thus the compositions of Examples 2 and 3 have a sustained-release characteristic. Particularly, the liposome formulation composition of Example 3 showed fluorescence intensities of similar values when 2 hours and 8 hours have elapsed, and thus it was confirmed that it stays inside the stratum corneum at a constant rate and has a continuous sustained-release mechanism.

Through this, it could be seen that a composition including the allantoin micro-needle crystal according to one aspect of the present disclosure, regardless of its type, penetrates into a skin stratum corneum after being applied to skin, and thereafter, allantoin, which is an active ingredient, is gradually released for a long time and continuously absorbed into the stratum corneum and an epidermis layer, thereby having a sustained-release characteristic.

### [Experimental Example 3] Confirmation of effect of allantoin micro-needle crystal as active ingredient delivery system

As can be seen from the results of the Experimental Examples 1 and 2, the allantoin micro-needle crystal according to one aspect of the present disclosure penetrates into skin and can continuously release allantoin in a sustained-release manner, and thus it was predicted that the allantoin micro-needle crystal can be utilized as an active ingredient delivery system, and an experiment was performed to confirm such an effect.

### [Experimental Example 3-1] Manufacturing of system for delivering oil-soluble active ingredient

After the allantoin powder of the Experimental Example 1 was completely dissolved in glycerin, water was added thereto to precipitate in the same manner as in the Experimental Example 1, and filtration and drying under reduced pressure were performed to manufacture an allantoin micro-needle crystal. Then, retinol (retinol, Retinol 10s (GLYCINE SOJA (SOYBEAN) OIL*RETINOL)) (BASF Company Ltd., Retinol 10 SU^{™}) was added to the dried crystal to be impregnated. Thereafter, this was dispersed and mixed, and then dried again to obtain micro-needle crystals in which retinol is impregnated (Example 4). Each component and a content thereof during the manufacturing process are as shown in Table 1 below.

### [Experimental Example 3-2] Manufacturing of system for delivering water-soluble active ingredient

The allantoin powder of the Experimental Example 1 and a Centella asiatica (*Centella asiatica* selec.) extract (ASIATICOSIDE*MADECASSIC ACID*ASIATIC ACID) (Givaudan Fragrances Shanghai LTD, CENTELLA ASIATICA TRITERP./C^{™}) were dissolved together in glycerin, and then water was added thereto to precipitate in the same manner as in the Experimental Example 1, and filtration and drying under reduced pressure were performed. Through the process, micro-needle crystals containing the Centella asiatica extract were obtained (Example 5). Each component and a content thereof during the manufacturing process are as shown in Table 1 below.

### [Experimental Example 3-3] Evaluation of solubility and sensation of allantoin micro-needle crystal including active ingredient

Solubility of the allantoin micro-needle crystal of Examples 4 and 5 in polyol, specifically glycerin, a crystal form, and a sensation when applying them to skin were evaluated and are shown in Table 1 below. In this case, as for the solubility, one that does not dissolve was indicated by X, one that slightly dissolves was indicated by △, and one that dissolves well was indicated by O, and the sensation was evaluated by whether a sensation to an extent of receiving a dermatological procedure is felt when Examples 4 and 5 are applied to a face.

**TABLE 1**

| Unit: wt% | | |
|---|---|---|
| | Example 4 | Example 5 |
| Allantoin | 6 | 6 |
| Urea | 2 | 2 |
| Betaine | 18 | 18 |
| Glycerin | 30 | 30 |
| Butylene glycol | 5 | 5 |
| Retinol | 1 | - |
| Centella asiatica extract (*Centella asiatica* selec.)(ASIATICOSIDE*MADECASSIC ACID*ASIATIC ACID) | - | 0.3 |
| Water | To 100 | To 100 |
| Total Sum | 100 | 100 |
| Solubility | O | O |
| Form | Acicular | Acicular |
| Sensation | O | O |

### [Experimental Example 3-4] Confirmation of effect of allantoin micro-needle crystal as active ingredient delivery system

In order to confirm whether the allantoin micro-needle crystal of Examples 4 and 5 can act as an active ingredient delivery system, an addition amount of each impregnated active ingredient in the Experimental Examples 3-1 and 3-2, a content within a finally manufactured crystal, and a ratio of the content within the crystal to the addition amount were measured, and results thereof are as shown in Table 2 below.

**TABLE 2**

| Example | Name of impregnated component | Addition amount | Content within crystal | Ratio of content within crystal to addition amount |
|---|---|---|---|---|
| Example 4 | Retinol | 33,000 (IU/g) | 31,079 IU/g | 94.2 (%) |
| Example 5 | Asiaticoside (40 wt% based on total weight of raw material) | 0.12 (%) | 0.080 (%) | 66.6 (%) |
| | Asiatic acid (26 wt% based on total weight of raw material) | 0.08 (%) | 0.048 (%) | 61.5 (%) |

As a result, it was confirmed that retinol (FIG. 6), which is a representative oil-soluble component, was completely impregnated into the allantoin micro-needle crystal such that an appearance of the retinol, which was white, changed to yellow, and was oil-absorbed into the crystal in a state where the content of retinol was maintained. FIG. 7 is a photograph of an allantoin micro-needle crystal in which retinol is impregnated, dissolved in deionized water. In addition, it was confirmed that for a Centella asiatica extract (FIG. 8), which is a representative component that is water-soluble, that is, dissolved in polyol, about 60 to 67% was co-crystallized to form the allantoin micro-needle crystal, and the remaining about 33 to 40% was dissolved and dispersed in water. For both of the components, homogeneity was % RSD < 2%.

Through this, it could be seen that the allantoin micro-needle crystal according to one aspect of the present disclosure can be utilized as an active ingredient delivery system that delivers various components, specifically, an oil-soluble or water-soluble active ingredient into skin by crystallizing allantoin, which was poorly soluble, by changing it to be soluble.

Therefore, the allantoin micro-needle crystal and a composition (regardless of type) comprising the same according to one aspect of the present disclosure have a sustained-release characteristic in which the allantoin is gradually released and continuously absorbed into a stratum corneum and an epidermis layer by penetrating into the skin, and can be utilized as an active ingredient delivery system for delivering various ingredients, specifically, an active ingredient that is oil-soluble or water-soluble into the skin, so that the allantoin itself or the active ingredient impregnated in the crystal is released in a sustained-release manner, whereby an effect lasts for a long time and a stable effect is maintained, side effects such as skin irritation or allergic reactions are reduced, and furthermore, the active ingredient can be continuously delivered to a specific site, which is effective for improving a skin condition, and since a small amount of ingredients is continuously released, there is an excellent effect that the skin can be easily managed for a long time without a burden on a skin barrier.

Hereinafter, formulation examples of a composition according to the present invention will be described, but this is not intended to limit the present invention, but is merely intended to describe the same in detail.

### [Formulation Example 1] Nourishing Cosmetic Water

Nourishing cosmetic water may be manufactured by a conventional method according to a composition described in Table 3 below.

**TABLE 3**

| Name of Raw material | Content (wt%) |
|---|---|
| Glycerin | 3.0 |
| Butylene glycol | 3.0 |
| Propylene glycol | 3.0 |
| Carboxyvinyl polymer | 0.1 |
| Allantoin micro-needle crystal of Example 1 | 0.1 |
| Beeswax | 4.0 |
| Polysorbate 60 | 1.5 |
| Caprylic/Capric triglyceride | 5.0 |
| Squalane | 5.0 |
| Sorbitan sesquioleate | 1.5 |
| Cetearyl alcohol | 1.0 |
| Triethanolamine | 0.2 |
| Antiseptic, fragrance | q.s. |
| Purified water | balance |
| Total | 100 |

### [Formulation Example 2] Nourishing Lotion

Nourishing lotion may be manufactured by a conventional method according to a composition described in Table 4 below.

**TABLE 4**

| Name of Raw material | Content (wt%) |
|---|---|
| Purified water | balance |
| Glycerin | 3.0 |
| Butylene glycol | 3.0 |
| Liquid paraffin | 5.0 |
| Beta-glucan | 7.0 |
| Carbomer | 0.1 |
| Allantoin micro-needle crystal of Example 1 | 10.0 |
| Caprylic/Capric triglyceride | 3.0 |
| Squalane | 5.0 |
| Cetearyl glucoside | 1.5 |
| Sorbitan stearate | 0.4 |
| Polysorbate 60 | 1.5 |
| Antiseptic | q.s. |
| Fragrance | q.s. |
| Colorant | q.s. |
| Triethanolamine | 0.1 |
| Total | 100 |

### [Formulation Example 3] Nourishing Cream

A nourishing cream may be manufactured by a conventional method according to the composition described in Table 5 below.

**TABLE 5**

| Name of Raw material | Content (wt%) |
|---|---|
| Glycerin | 3.5 |
| Butylene glycol | 3.0 |
| Liquid paraffin | 7.0 |
| Beta-glucan | 7.0 |
| Carbomer | 0.1 |
| Allantoin micro-needle crystal of Example 1 | 10.0 |
| Caprylic/Capric triglyceride | 3.0 |
| Squalane | 5.0 |
| Cetearyl glucoside | 1.5 |
| Sorbitan stearate | 0.4 |
| Polysorbate 60 | 1.2 |
| Triethanolamine | 0.1 |
| Antiseptic, fragrance | q.s. |
| Purified water | balance |
| Total | 100 |

### [Formulation Example 4] Ointment

Ointment may be manufactured by a conventional method according to a composition described in Table 6 below.

**TABLE 6**

| Name of Raw material | Content (wt%) |
|---|---|
| Glycerin | 8.0 |
| Butylene glycol | 4.0 |
| Liquid paraffin | 15.0 |
| Beta-glucan | 7.0 |
| Carbomer | 0.1 |
| Allantoin micro-needle crystal of Example 1 | 0.1 |
| Caprylic/Capric triglyceride | 3.0 |
| Squalane | 1.0 |
| Cetearyl glucoside | 1.5 |
| Sorbitan stearate | 0.4 |
| Cetearyl alcohol | 1.0 |
| Antiseptic | q.s. |
| Fragrance | q.s. |
| Colorant | q.s. |
| Beeswax | 4.0 |
| Purified water | balance |

In view of the foregoing, specific portions of the content of the present invention have been described in detail, and to a person having ordinary skill in the art, such specific descriptions are merely a preferable embodiment, and it will be apparent that the scope of the present invention is not limited thereby. Therefore, the substantial scope of the present invention is defined by the appended claims and their equivalents.

## Claims

1. A transdermal administration composition of a sustained-release formulation comprising an allantoin micro-needle crystal.

2. The transdermal administration composition of claim 1, wherein the allantoin micro-needle crystal has one or more features of:
an average length of a long axis of 5 to 500 µm; and
a thickness of greater than 0 and 40 µm or less.

3. The transdermal administration composition of claim 1 or 2, wherein the allantoin micro-needle crystal is soluble.

4. The transdermal administration composition of any one of claims 1 to 3, wherein the allantoin micro-needle crystal penetrates into skin when applied to the skin.

5. The transdermal administration composition of any one of claims 1 to 4, wherein the allantoin micro-needle crystal exists inside a skin stratum corneum when 2 to 36 hours have elapsed after being applied to skin.

6. The transdermal administration composition of any one of claims 1 to 5, wherein the allantoin micro-needle crystal is dissolved and absorbed in skin when repeatedly rolled 10 times with a weight of 0.5 to 1.0 kg/cm² after being applied to the skin.

7. The transdermal administration composition of any one of claims 1 to 6, wherein the allantoin micro-needle crystal further comprises betaine.

8. The transdermal administration composition of any one of claims 1 to 7, wherein the transdermal administration composition is a cosmetic composition.

9. A use of the transdermal administration composition of any one of claims 1 to 7 for exfoliation, skin moisturizing, skin wound healing, promoting skin regeneration, or skin protection against irritation.

10. The transdermal administration composition of any one of claims 1 to 7, for use in skin wound healing.

11. The transdermal administration composition of any one of claims 1 to 8 and 10, wherein the transdermal administration composition is a composition of an oil dispersion, a water dispersion, a water-in-oil, an oil-in-water, or a multiple emulsion type.

12. An active ingredient delivery system for delivering an active ingredient into skin, wherein the active ingredient system comprises an allantoin micro-needle crystal, and the allantoin micro-needle crystal has one or more features of:
an average length of a long axis of 5 to 500 µm; and
a thickness of greater than 0 µm and 40 µm or less.

13. The active ingredient delivery system of claim 12, wherein the active ingredient delivery system further comprises an active ingredient within the allantoin micro-needle crystal.

14. The active ingredient delivery system of claim 12 or 13, wherein the active ingredient is water-soluble or oil-soluble.
